# EUROPEAN PATENT APPLICATION

(11) **EP 4 609 800 A2**
(43) Date of publication of application: **03.09.2025**
(21) Application number: 25190016.3
(22) Date of filing: 02.06.2020
(51) Int. Cl.: A61B 8/00

(54) **ULTRASONIC DIAGNOSTIC DEVICE AND METHOD FOR CONTROLLING ULTRASONIC DIAGNOSTIC DEVICE**

(30) Priority: 23.07.2019 JP 2019135374
(62) Divisional of application: 24201487.6
(71) Applicant: FUJI-FILM Corporation, Minato-ku Tokyo 106-8620 (JP)
(72) Inventor: MATSUMOTO, Tsuyoshi, Kanagawa (JP); IMAI, Yoshiro, Kanagawa (JP); TASHIRO, Rika, Kanagawa (JP)
(74) Representative: Dehns Germany Partnerschaft mbB

(57) **Abstract**

An ultrasound diagnostic apparatus that displays a blood vessel of a subject on an ultrasound image, the ultrasound diagnostic apparatus comprising: a transducer array (2); a transmission and reception circuit configured to cause the transducer array to transmit an ultrasound beam toward the subject, and to process a reception signal output from the transducer array that has received an ultrasound echo from the subject to generate a sound ray signal; an image generation unit (6) configured to generate the ultrasound image on the basis of the sound ray signal generated by the transmission and reception circuit; a display device (8) arranged to display the ultrasound image generated by the image generation unit; a blood vessel extraction unit (9) configured to extract blood vessel regions by analyzing the ultrasound image generated by the image generation unit; a highlight region setting unit (11) configured to set at least one highlight region extending in a depth direction of the ultrasound image; a highlighting unit (12) configured to highlight the blood vessel region which is extracted by the blood vessel extraction unit and overlaps the at least one highlight region set by the highlight region setting unit, on the display device; and an insertion object path estimation unit (41) configured to detect an insertion object and to estimate a path of the detected insertion object by analyzing the ultrasound image generated by the image generation unit in a case where the insertion object is inserted into the blood vessel of the subject, wherein in a case where a plurality of the blood vessel regions each overlapping the highlight region are detected by the blood vessel extraction unit and the insertion object is detected by the insertion object path estimation unit, the highlighting unit performs highlighting such that the blood vessel region overlapping both the highlight region and the path of the insertion object estimated by the insertion object path estimation unit, and the blood vessel region overlapping only the highlight region have different colors or brightness from each other.

## Description

### BACKGROUND OF THE INVENTION

### 1. Field of the Invention

The present invention relates to an ultrasound diagnostic apparatus, and a control method of the ultrasound diagnostic apparatus which display a blood vessel of a subject on an ultrasound image.

### 2. Description of the Related Art

In the related art, an ultrasound diagnostic apparatus has been known as an apparatus for obtaining an image of the inside of a subject. The ultrasound diagnostic apparatus generally comprises an ultrasound probe comprising a transducer array in which a plurality of ultrasonic transducers are arranged. In a state where the ultrasound probe is in contact with the body surface of the subject, an ultrasound beam is transmitted toward the inside of the subject from the transducer array and an ultrasound echo from the subject is received by the transducer array so that an electric signal corresponding to the ultrasound echo is acquired. Further, the ultrasound diagnostic apparatus electrically processes the obtained electric signal to generate an ultrasound image of the corresponding site of the subject.

By the way, a procedure of inserting an insertion object such as a so-called puncture needle and a catheter into the blood vessel of the subject while observing the inside of the subject using the ultrasound diagnostic apparatus is known. In this manner, in a case where the insertion object is inserted into the blood vessel of the subject, normally, an operator needs to grasp the position and shape of the blood vessel included in the ultrasound image by checking the generated ultrasound image, but in order to accurately grasp the position and shape of the blood vessel, a skill level equal to or more than a certain level is required. Therefore, for example, as disclosed in JP2017-524455A, an ultrasound diagnostic apparatus has been developed which automatically detects a blood vessel included in an ultrasound image, measures a diameter and an area of the detected blood vessel, and causes a display device to display the measured diameter and area of the blood vessel.

### SUMMARY OF THE INVENTION

In the ultrasound diagnostic apparatus in JP2017-524455A, in a case where the blood vessel is detected, even if the detected blood vessel is not the blood vessel that is not the observation target of the operator, the diameter and the area of the blood vessel are displayed on the display device. Therefore, for the operator with a low skill level, the difference of the blood vessel included in the ultrasound image is not clear, and it is difficult for the operator to accurately grasp the blood vessel as the observation target by checking the ultrasound image in some cases.

The present invention has been made in order to solve such a problem in the related art, and an object thereof is to provide an ultrasound diagnostic apparatus which allows an operator to intuitively and accurately grasp the blood vessel as the observation target in the ultrasound image, and a control method of the ultrasound diagnostic apparatus.

In order to achieve the object, an ultrasound diagnostic apparatus according to an aspect of the present invention is an ultrasound diagnostic apparatus that displays a blood vessel of a subject on an ultrasound image, the ultrasound diagnostic apparatus comprising: a transducer array; a transmission and reception circuit configured to cause the transducer array to transmit an ultrasound beam toward the subject, and to process a reception signal output from the transducer array that has received an ultrasound echo from the subject to generate a sound ray signal; an image generation unit configured to generate the ultrasound image on the basis of the sound ray signal generated by the transmission and reception circuit; a display device arranged to display the ultrasound image generated by the image generation unit; a blood vessel extraction unit configured to extract blood vessel regions by analyzing the ultrasound image generated by the image generation unit; a highlight region setting unit configured to set at least one highlight region extending in a depth direction of the ultrasound image; a highlighting unit configured to highlight the blood vessel region which is extracted by the blood vessel extraction unit and overlaps the at least one highlight region set by the highlight region setting unit, on the display device; and an insertion object path estimation unit configured to detect an insertion object and to estimate a path of the detected insertion object by analyzing the ultrasound image generated by the image generation unit in a case where the insertion object is inserted into the blood vessel of the subject, wherein in a case where a plurality of the blood vessel regions each overlapping the highlight region are detected by the blood vessel extraction unit and the insertion object is detected by the insertion object path estimation unit, the highlighting unit performs highlighting such that the blood vessel region overlapping both the highlight region and the path of the insertion object estimated by the insertion object path estimation unit, and the blood vessel region overlapping only the highlight region have different colors or brightness from each other.

The ultrasound diagnostic apparatus may further comprise a blood vessel information acquisition unit that acquires blood vessel information including at least one of a diameter or a depth of the blood vessel region overlapping the highlight region by analyzing the ultrasound image generated by the image generation unit, and in this case, it is preferable that the highlighting unit displays the blood vessel information acquired by the blood vessel information acquisition unit on the display device in association with the blood vessel region overlapping the highlight region.

The blood vessel information acquisition unit may further acquire information representing whether the blood vessel region belongs to an artery or a vein, as the blood vessel information.

In a case where a plurality of the blood vessel regions are extracted by the blood vessel extraction unit, the highlighting unit may highlight each of the plurality of blood vessel regions extracted by the blood vessel extraction unit on the display device, and highlight, among the plurality of blood vessel regions, the blood vessel region overlapping the highlight region and the blood vessel region not overlapping the highlight region in different formats.

In this case, the highlighting unit may perform highlighting such that, among the plurality of blood vessel regions extracted by the blood vessel extraction unit, the blood vessel region overlapping the highlight region and the blood vessel region not overlapping the highlight region have different colors or brightness from each other.

In a case where a plurality of the blood vessel regions each overlapping the highlight region are extracted by the blood vessel extraction unit, the highlighting unit may perform highlighting such that the plurality of blood vessel regions extracted by the blood vessel extraction unit have different colors or brightness from each other according to a depth or size of the blood vessel regions.

The ultrasound diagnostic apparatus may further comprise an ultrasound probe including the transducer array; and a probe motion detection unit that detects a motion of the ultrasound probe, and determines whether the ultrasound probe is stationary on the basis of the detected motion of the ultrasound probe, and in this case, the highlighting unit may display the blood vessel information acquired by the blood vessel information acquisition unit on the display device only in a case where the probe motion detection unit determines that the ultrasound probe is stationary.

The ultrasound diagnostic apparatus may further comprise a device control unit that sets an imaging condition such that the blood vessel region is clearly depicted in the ultrasound image, and controls the transmission and reception circuit and the image generation unit on the basis of the set imaging condition, in a case where the blood vessel region extracted by the blood vessel extraction unit overlaps the highlight region.

The highlight region setting unit may display the set highlight region on the display device.

In this case, the highlight region setting unit may display only a part of the highlight region, which does not overlap the blood vessel region, on the display device in a case where the blood vessel region extracted by the blood vessel extraction unit overlaps the highlight region.

A control method of an ultrasound diagnostic apparatus is also provided as defined by claim 9.

According to the present invention, since there are provided a blood vessel extraction unit that extracts a blood vessel region by analyzing an ultrasound image generated by an image generation unit; a highlight region setting unit that sets at least one highlight region extending in a depth direction of the ultrasound image; and a highlighting unit that highlights the blood vessel region which is extracted by the blood vessel extraction unit and overlaps the at least one highlight region set by the highlight region setting unit, on a display device, an operator can intuitively and accurately grasp a blood vessel as an observation target in the ultrasound image.

### BRIEF DESCRIPTION OF THE DRAWINGS

Fig. 1 is a block diagram illustrating a configuration of an ultrasound diagnostic apparatus according to a first embodiment of the present invention.
Fig. 2 is a block diagram illustrating an internal configuration of a reception circuit in the first embodiment of the present invention.
Fig. 3 is a block diagram illustrating an internal configuration of an image generation unit in the first embodiment of the present invention.
Fig. 4 is a schematic diagram of an ultrasound image indicating a state in which a blood vessel region overlapping a highlight region is highlighted, in the first embodiment of the present invention.
Fig. 5 is a flowchart illustrating an operation of the ultrasound diagnostic apparatus according to the first embodiment of the present invention.
Fig. 6 is a schematic diagram of an ultrasound image indicating a state in which two blood vessel regions overlap the same highlight region, in the first embodiment of the present invention.
Fig. 7 is a diagram illustrating an example of an ultrasound probe according to the first embodiment of the present invention.
Fig. 8 is a schematic diagram of an ultrasound image in which only a part of the highlight region, which does not overlap the blood vessel region, is displayed, in the first embodiment of the present invention.
Fig. 9 is a schematic diagram of an ultrasound image including a gel layer and a body surface of a subject and indicating a state in which a blood vessel region overlapping a highlight region is highlighted.
Fig. 10 is a schematic diagram of an ultrasound image including a gel layer and a body surface of a subject and indicating a state in which all blood vessel regions are highlighted and blood vessel information for the blood vessel region overlapping a highlight region is displayed.
Fig. 11 is a schematic diagram illustrating a highlight region in a case of using a convex type ultrasound probe.
Fig. 12 is a block diagram illustrating a configuration of an ultrasound diagnostic apparatus according to a second embodiment of the present invention.
Fig. 13 is a schematic diagram of an ultrasound image including a blood vessel overlapping both a path of an insertion object to be inserted into the blood vessel of the subject and the highlight region, in the second embodiment of the present invention.
Fig. 14 is a block diagram illustrating a configuration of an ultrasound diagnostic apparatus according to a third embodiment of the present invention.
Fig. 15 is a block diagram illustrating a configuration of an ultrasound diagnostic apparatus according to a fourth embodiment of the present invention.

### DESCRIPTION OF THE PREFERRED EMBODIMENTS

Hereinafter, embodiments of the invention will be described with reference to the accompanying drawings.

The description of configuration requirements described below is given on the basis of the representative embodiment of the present invention, but the present invention is not limited to such an embodiment.

In the present specification, a numerical range represented using "to" means a range including the numerical values before and after "to" as a lower limit value and an upper limit value.

In addition, in the present specification, the terms "perpendicular" and "parallel" include a range of errors allowed in the technical field to which the present invention belongs. For example, the terms "perpendicular" and "parallel" mean a range less than ±10 degrees with respect to the strict perpendicular or parallel, and the error with respect to the strict perpendicular or parallel is preferably 5 degrees or less, and more preferably 3 degrees or less.

In the present specification, the terms "same" and "identical" include an error range generally allowed in the technical field. Further, in the present specification, in a case of referring to "all", "any", or "whole surface", the term includes an error range generally allowed in the technical field in addition to a case of 100%, and includes, for example, a case of 99% or more, a case of 95% or more, or a case of 90% or more.

### First Embodiment

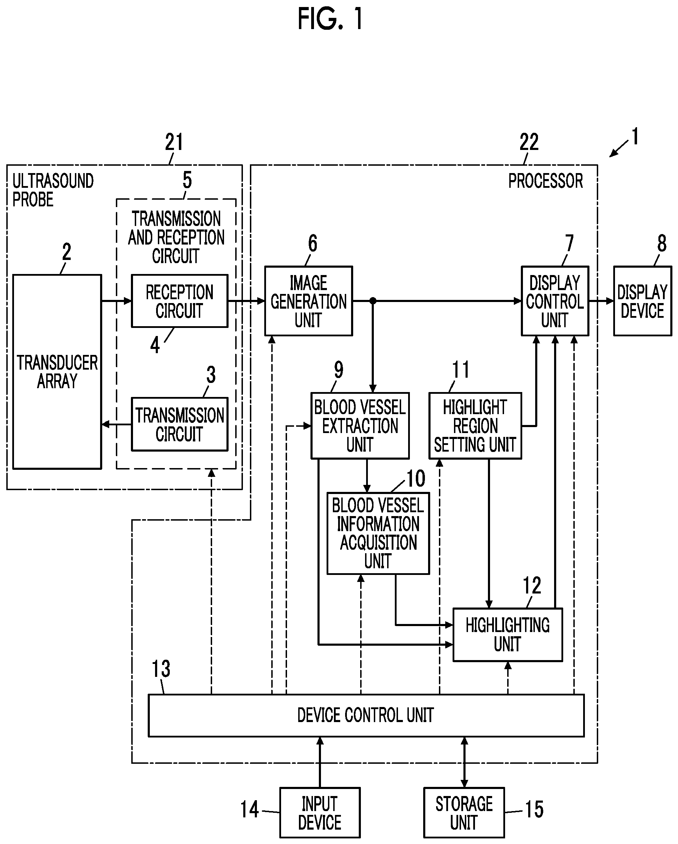

Fig. 1 illustrates a configuration of an ultrasound diagnostic apparatus 1 according to a first embodiment of the present invention. The ultrasound diagnostic apparatus 1 comprises a transducer array 2, and each of a transmission circuit 3 and a reception circuit 4 is connected to the transducer array 2. Here, the transmission circuit 3 and the reception circuit 4 constitute a transmission and reception circuit 5, and an image generation unit 6, a display control unit 7, and a display device 8 are sequentially connected to the reception circuit 4. A blood vessel extraction unit 9 is connected to the image generation unit 6. A blood vessel information acquisition unit 10 is connected to the blood vessel extraction unit 9. The ultrasound diagnostic apparatus 1 has a highlight region setting unit 11, and a highlighting unit 12 is connected to the blood vessel extraction unit 9, the blood vessel information acquisition unit 10, and the highlight region setting unit 11. The highlight region setting unit 11 and the highlighting unit 12 are connected to the display control unit 7.

In addition, a device control unit 13 is connected to the transmission and reception circuit 5, the image generation unit 6, the display control unit 7, the blood vessel extraction unit 9, the blood vessel information acquisition unit 10, the highlight region setting unit 11, and the highlighting unit 12, and an input device 14 and a storage unit 15 are connected to the device control unit 13. The device control unit 13 and the storage unit 15 are connected so as to exchange information bidirectionally.

The transducer array 2 and the transmission and reception circuit 5 are included in an ultrasound probe 21. Further, the image generation unit 6, the display control unit 7, the blood vessel extraction unit 9, the blood vessel information acquisition unit 10, the highlight region setting unit 11, the highlighting unit 12, and the device control unit 13 constitute a processor 22 for the ultrasound diagnostic apparatus 1.

The transducer array 2 of the ultrasound probe 21 illustrated in Fig. 1 has a plurality of transducers arranged in a one-dimensional or two-dimensional manner. According to a drive signal supplied from the transmission circuit 3, each of the transducers transmits an ultrasonic wave and receives an ultrasound echo from a subject to output a signal based on the ultrasound echo. For example, each transducer is configured by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

The transmission circuit 3 includes, for example, a plurality of pulse generators, and the transmission circuit 3 adjusts the amount of delay of each drive signal so that ultrasonic waves transmitted from the plurality of transducers of the transducer array 2 form an ultrasound beam on the basis of a transmission delay pattern selected according to the control signal from the device control unit 13, and supplies the obtained signals to the plurality of transducers. Thus, in a case where a pulsed or continuous-wave voltage is applied to the electrodes of the transducers of the transducer array 2, the piezoelectric body expands and contracts to generate pulsed or continuous-wave ultrasonic waves from each transducer. From the combined wave of these ultrasonic waves, an ultrasound beam is formed.

The transmitted ultrasound beam is reflected by a target, for example, a site of the subject, and propagates toward the transducer array 2 of the ultrasound probe 21. The ultrasound echo propagating toward the transducer array 2 in this manner is received by each transducer constituting the transducer array 2. In this case, each transducer constituting the transducer array 2 expands and contracts by receiving the propagating ultrasound echo to generate electric signals, and outputs the electric signals to the reception circuit 4.

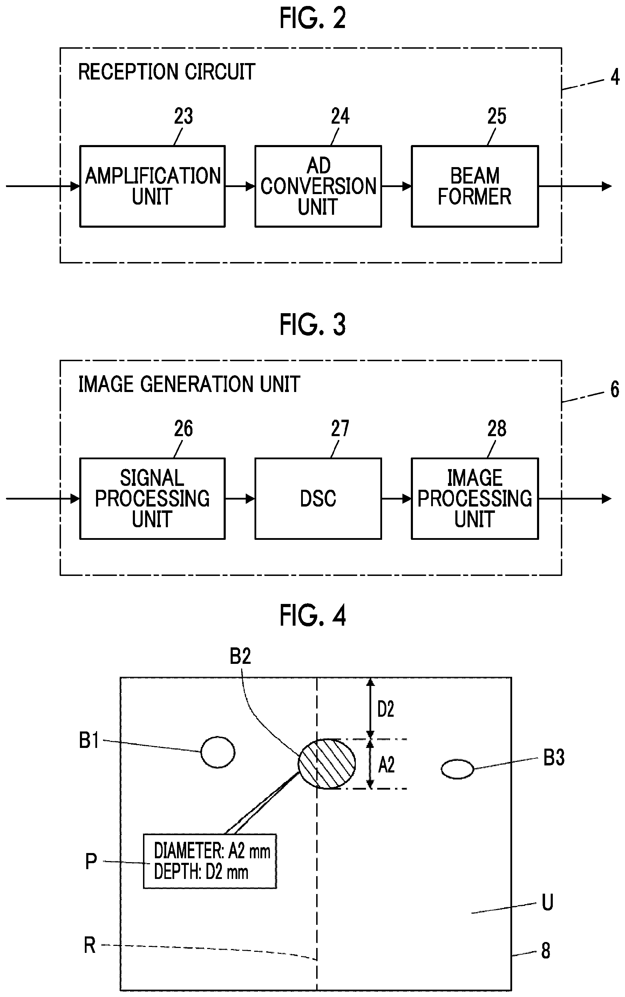

The reception circuit 4 processes the signal output from the transducer array 2 according to the control signal from the device control unit 13, and generates a sound ray signal. As illustrated in Fig. 2, the reception circuit 4 has a configuration in which an amplification unit 23, an analog digital (AD) conversion unit 24, and a beam former 25 are connected in series.

The amplification unit 23 amplifies the signal input from each transducer constituting the transducer array 2, and transmits the amplified signal to the AD conversion unit 24. The AD conversion unit 24 converts the signal transmitted from the amplification unit 23 into digital reception data, and transmits the reception data to the beam former 25. The beam former 25 performs so-called reception focusing processing in which addition is performed by giving delays to respective pieces of the reception data converted by the AD conversion unit 24 according to a sound speed distribution or a sound speed set on the basis of a reception delay pattern selected according to the control signal from the device control unit 13. Through the reception focusing processing, a sound ray signal in which each piece of the reception data converted by the AD conversion unit 24 is phased and added and the focus of the ultrasound echo is narrowed is acquired.

As illustrated in Fig. 3, the image generation unit 6 has a configuration in which a signal processing unit 26, a digital scan converter (DSC) 27, and an image processing unit 28 are sequentially connected in series.

The signal processing unit 26 generates a B-mode image signal, which is tomographic image information regarding tissues inside the subject, by performing, on the sound ray signal generated by the reception circuit 4, correction of the attenuation due to the distance according to the depth of the reflection position of the ultrasonic wave and then performing envelope detection processing.

The DSC 27 converts (raster conversion) the B-mode image signal generated by the signal processing unit 26 into an image signal according to a normal television signal scanning method.

The image processing unit 28 performs various kinds of necessary image processing such as gradation processing on the B-mode image signal input from the DSC 27, and then outputs the B-mode image signal to the display control unit 7 and the blood vessel extraction unit 9. In the following, the B-mode image signal subjected to the image processing by the image processing unit 28 is simply referred to as an ultrasound image.

The blood vessel extraction unit 9 extracts blood vessel regions B1, B2, and B3 included in an ultrasound image U, for example, as illustrated in Fig. 4 by analyzing the ultrasound image generated by the image generation unit 6. Here, in Fig. 4, the blood vessel regions B1, B2, and B3 corresponding to the cross sections of three blood vessels are exemplified. Here, the cross section of the blood vessel refers to a cut section of the blood vessel along a direction orthogonal to a traveling direction of the blood vessel.

The blood vessel extraction unit 9 can extract the blood vessel regions B1, B2, and B3 in the ultrasound image U using a known algorithm. For example, the blood vessel extraction unit 9 can store typical pattern data of the blood vessel region in advance as a template, calculate a similarity degree for the pattern data while searching the ultrasound image U using the template, and consider that the blood vessel regions B1, B2, and B3 are present in places where the similarity degree is equal to or greater than a threshold value and is the maximum.

Further, for the calculation of the similarity degree, in addition to simple template matching, for example, a machine learning method described in Csurka et al.: Visual Categorization with Bags of Keypoints, Proc. of ECCV Workshop on Statistical Learning in Computer Vision, pp. 59-74 (2004) or a general image recognition method using deep learning described in Krizhevsk et al.: ImageNet Classification with Deep Convolutional Neural Networks, Advances in Neural Information Processing Systems 25, pp.1106-1114 (2012) can be used.

The highlight region setting unit 11 sets at least one highlight region extending in a depth direction of the ultrasound image U. For example, the highlight region setting unit 11 can set a linear highlight region R extending along the depth direction of the ultrasound image U as illustrated in Fig. 4. In the example illustrated in Fig. 4, one linear highlight region R is disposed at the center in a direction orthogonal to the depth direction of the ultrasound image U, but is not particularly limited thereto. For example, two or more highlight regions R may be set, the shape of the highlight region R may not be a linear shape, and a placement position of the highlight region can be arbitrarily set.

The highlight region setting unit 11 can set the highlight region R according to information relating to the shape, placement position, number, and the like of the highlight region R, which is input by the operator through the input device 14 in advance, for example.

The blood vessel information acquisition unit 10 acquires blood vessel information including a diameter and a depth of the blood vessel regions B1, B2, and B3 by analyzing the ultrasound image U generated by the image generation unit 6. For example, as illustrated in Fig. 4, the blood vessel information acquisition unit 10 can measure the width of the blood vessel region B2 in the depth direction of the ultrasound image U, as a diameter A2 of the blood vessel region B2. For example, the blood vessel information acquisition unit 10 can measure the shallowest position of the ultrasound image U, that is, the shortest distance from the upper end portion of the ultrasound image U to the blood vessel region B2 in the depth direction, as a depth D2 of the blood vessel region B2.

As illustrated in Fig. 4, the highlighting unit 12 highlights the blood vessel region B2 which is one of the blood vessel regions B1, B2, and B3 extracted by the blood vessel extraction unit 9 and overlaps the highlight region R set by the highlight region setting unit 11, on the display device 8. For example, the highlighting unit 12 can display the blood vessel region B2 on the display device 8 such that the blood vessel region B2 has a different color, brightness, or the like from the other regions in the ultrasound image U. Further, the highlighting unit 12 can display the blood vessel information acquired by the blood vessel information acquisition unit 10 on the display device 8 in association with the blood vessel region B2 overlapping the highlight region R, as the highlighting for the blood vessel region B2. In the example illustrated in Fig. 4, a blood vessel information panel P including the blood vessel information corresponding to the blood vessel region B2 is superimposed on the ultrasound image U to be displayed on the display device 8.

The device control unit 13 controls each unit of the ultrasound diagnostic apparatus 1 on the basis of a program stored in advance in the storage unit 15 or the like and the operator's input operation through the input device 14.

The display control unit 7 performs predetermined processing on the ultrasound image U generated by the image generation unit 6 and displays the ultrasound image U on the display device 8, under the control of the device control unit 13.

The display device 8 is for displaying the ultrasound image U and the like under the control of the display control unit 7, and includes a display device such as a liquid crystal display (LCD), or an organic electroluminescence (EL) display.

The input device 14 is for the operator to perform an input operation, and can be configured to comprise a keyboard, a mouse, a trackball, a touchpad, a touch panel, and the like.

The storage unit 15 stores a control program and the like of the ultrasound diagnostic apparatus 1, and recording media such as a flash memory, a hard disk drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a universal serial bus memory (USB memory), a server, or the like can be used.

The processor 22 having the image generation unit 6, the display control unit 7, the blood vessel extraction unit 9, the blood vessel information acquisition unit 10, the highlight region setting unit 11, the highlighting unit 12, and the device control unit 13 is configured by a central processing unit (CPU) and a control program for causing the CPU to execute various kinds of processing, but the processor 22 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the image generation unit 6, the display control unit 7, the blood vessel extraction unit 9, the blood vessel information acquisition unit 10, the highlight region setting unit 11, the highlighting unit 12, and the device control unit 13 of the processor 22 can also be configured by being integrated partially or entirely into one CPU or the like.

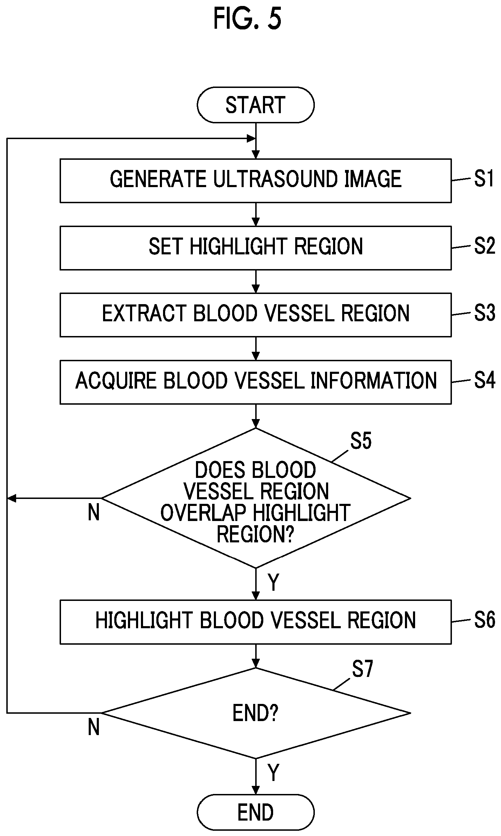

In the following, the operation of the ultrasound diagnostic apparatus 1 in the first embodiment will be described in detail using the flowchart illustrated in Fig. 5.

In Step S1, the ultrasound image U is generated, and the generated ultrasound image U is displayed on the display device 8. In this case, the ultrasound probe 21 is brought into contact with the body surface of the subject by the operator, an ultrasound beam is transmitted into the subject from the plurality of transducers of the transducer array 2 according to the drive signal from the transmission circuit 3, and the reception signal is output to the reception circuit 4 from each transducer which has received the ultrasound echo from the subject. The reception signal received by the reception circuit 4 is amplified in the amplification unit 23, is subjected to the AD conversion in the AD conversion unit 24, and is phased and added in the beam former 25, and thereby the sound ray signal is generated. The sound ray signal is subjected to the envelope detection processing by the signal processing unit 26 to become the B-mode image signal in the image generation unit 6, and is output to the display control unit 7 via the DSC 27 and the image processing unit 28, and the ultrasound image U is displayed on the display device 8 under the control of the display control unit 7 as illustrated in Fig. 4. In the example illustrated in Fig. 4, the generated ultrasound image U includes three blood vessel regions B1, B2, and B3.

Next, in Step S2, the highlight region setting unit 11 sets at least one highlight region R extending in the depth direction of the ultrasound image U. For example, the highlight region setting unit 11 can set the linear highlight region R extending along the depth direction of the ultrasound image U as illustrated in Fig. 4. In this case, the highlight region setting unit 11 can set the highlight region R according to information relating to the shape, placement position, number, and the like of the highlight region R, which is input by the operator through the input device 14 in advance, for example.

In Step S3, the blood vessel extraction unit 9 extracts the blood vessel regions B1, B2, and B3 included in the ultrasound image U, for example, as illustrated in Fig. 4 by analyzing the ultrasound image U generated in Step S1. In this case, the blood vessel extraction unit 9 can extract the blood vessel regions B1, B2, and B3 by using, for example, a known algorithm such as template matching, a machine learning method, a general image recognition method using deep learning or the like.

In Step S4, the blood vessel information acquisition unit 10 acquires the blood vessel information including the diameter and depth of each of the blood vessel regions B1, B2, and B3 by analyzing the ultrasound image U generated in Step S1. For example, as illustrated in Fig. 4, the blood vessel information acquisition unit 10 can measure the width of the blood vessel region B2 in the depth direction of the ultrasound image U, as the diameter A2 of the blood vessel region B2. For example, the blood vessel information acquisition unit 10 can measure the shallowest position of the ultrasound image U, that is, the shortest distance from the upper end portion of the ultrasound image U to the blood vessel region B2 in the depth direction, as the depth D2 of the blood vessel region B2.

In Step S5, the highlighting unit 12 determines whether the highlight region R set in Step S2 overlaps any one of the blood vessel region B1, B2, or B3 extracted in Step S3, in the ultrasound image U generated in Step S1. Here, in a case where it is determined that the highlight region R does not overlap any one of the blood vessel region B1, B2, or B3, the processing proceeds to Step S1, and the ultrasound image U is newly generated. In subsequent Step S2, the highlight region R is set on the ultrasound image U newly generated in Step S1, the blood vessel regions B1, B2, and B3 are extracted in Step S3, the blood vessel information relating to the blood vessel regions B1, B2, and B3 is acquired in Step S4, and the processing proceeds to Step S5.

In Step S5, in a case where it is determined that the highlight region R overlaps any one of the blood vessel region B1, B2, or B3, the processing proceeds to Step S6. Here, in the example illustrated in Fig. 4, the linear highlight region R overlaps the blood vessel region B2. In such a case, it is determined in Step S5 that the highlight region R overlaps the blood vessel region B2, and the processing proceeds to Step S6.

In Step S6, the highlighting unit 12 highlights the blood vessel region B2 determined to overlap the highlight region R in Step S5, on the display device 8. For example, the highlighting unit 12 can display the blood vessel region B2 on the display device 8 such that the blood vessel region B2 has a different color or brightness from the other regions in the ultrasound image U. Further, the highlighting unit 12 can display the blood vessel information acquired by the blood vessel information acquisition unit 10 on the display device 8 in association with the blood vessel region B2 overlapping the highlight region R, as the highlighting for the blood vessel region B2. In the example illustrated in Fig. 4, the blood vessel information panel P including the blood vessel information corresponding to the blood vessel region B2 is superimposed on the ultrasound image U to be displayed on the display device 8. In this manner, since the blood vessel region B2 overlapping the highlight region R is highlighted on the display device 8, the operator can intuitively and accurately grasp the blood vessel region B2 as the observation target by checking the ultrasound image U.

In subsequent Step S7, it is determined whether to end the operation of the ultrasound diagnostic apparatus 1. For example, in a case where an instruction to end the operation of the ultrasound diagnostic apparatus 1 is input by the operator through the input device 14 or the like, it is determined that the operation of the ultrasound diagnostic apparatus 1 is to be ended, and in a case where an instruction to end the operation of the ultrasound diagnostic apparatus 1 is not input, it is determined that the operation of the ultrasound diagnostic apparatus 1 is not to be ended. In a case where it is determined that the operation of the ultrasound diagnostic apparatus 1 is not to be ended, the processing returns to Step S1, and the ultrasound image U is newly generated. In a case where it is determined that the operation of the ultrasound diagnostic apparatus 1 is to be ended, the operation of the ultrasound diagnostic apparatus 1 is ended.

As described above, with the ultrasound diagnostic apparatus 1 according to the first embodiment of the present invention, since the highlight region R is set on the generated ultrasound image U and the blood vessel region B2 overlapping the highlight region R is highlighted on the display device 8, the operator can intuitively and accurately grasp the blood vessel region B2 as the observation target by checking the ultrasound image U.

In general, a procedure of inserting an insertion object such as a so-called puncture needle and a catheter into the blood vessel of the subject while observing the inside of the subject using the ultrasound diagnostic apparatus is known. With the ultrasound diagnostic apparatus 1 according to the first embodiment of the present invention, for example, in a case where the operator inserts the insertion object such as a so-called puncture needle and a catheter into the blood vessel corresponding to the blood vessel region B2 while observing the blood vessel region B2 of the subject, the operator can accurately grasp the blood vessel as a target into which the insertion object is inserted, and therefore it is possible to improve the accuracy of inserting the insertion object into the blood vessel.

Further, since the highlighting unit 12 displays the blood vessel information relating to the blood vessel region B2 overlapping the highlight region R, on the display device 8, the operator can grasp the diameter A2 and the depth D2 of the blood vessel corresponding to the blood vessel region B2, and therefore it is possible to improve the accuracy of inserting the insertion object into the blood vessel.

The blood vessel information acquisition unit 10 acquires the blood vessel information of all the blood vessel regions B1, B2, and B3 extracted by the blood vessel extraction unit 9, but can acquire the blood vessel information of only the blood vessel region B2 overlapping the highlight region R.

Further, an example in which the blood vessel information acquisition unit 10 acquires the diameter and depth of each of the blood vessel regions B1, B2, and B3 has been described, but the blood vessel information acquisition unit 10 can acquire only the diameter or can acquire only the depth among the diameter and depth of each of the blood vessel regions B1, B2, and B3.

In the example illustrated in Fig. 4, the blood vessel region B2 overlapping the highlight region R is displayed on the display device 8 so as to have a different color or brightness from other regions in the ultrasound image U, and the blood vessel information panel P representing the blood vessel information relating to the blood vessel region B2 is displayed on the display device 8. However the method of highlighting the blood vessel region B2 overlapping the highlight region R is not limited thereto. For example, the highlighting unit 12 can display the blood vessel region B2 on the display device 8 such that the blood vessel region B2 has a different color or brightness from the other regions in the ultrasound image U, without displaying the blood vessel information panel P on the display device 8.

For example, the highlighting unit 12 can display the blood vessel information relating to only the blood vessel region B2 overlapping the highlight region R among the blood vessel regions B1, B2, and B3 extracted by the blood vessel extraction unit 9, on the display device 8, instead of displaying the blood vessel region B2 on the display device 8 such that the blood vessel region B2 has the same color or brightness as the other regions in the ultrasound image U.

For example, the highlighting unit 12 can highlight the blood vessel region B2 by displaying a closed frame line surrounding the blood vessel region B2 such as the contour line of the blood vessel region B2, an arrow pointing to the blood vessel region B2, and the like on the display device 8, instead of displaying the blood vessel region B2 on the display device 8 such that the blood vessel region B2 has the same color or brightness as the other regions in the ultrasound image U.

The highlighting unit 12 can display all the blood vessel regions B1, B2, and B3 extracted by the blood vessel extraction unit 9, on the display device 8 such that the blood vessel regions B1, B2, and B3 have a different color or brightness from the other regions in the ultrasound image U. In this case, the highlighting unit 12 can highlight the blood vessel region B2 and the blood vessel regions B1 and B3 on the display device 8 in different formats so that the blood vessel region B2 overlapping the highlight region R is distinguished from the blood vessel regions B1 and B3 not overlapping the highlight region R. For example, the highlighting unit 12 can perform highlighting such that, among the blood vessel regions B1, B2, and B3, the blood vessel region B2 overlapping the highlight region R and the blood vessel regions B1 and B3 not overlapping the highlight region R have different colors or brightness from each other.

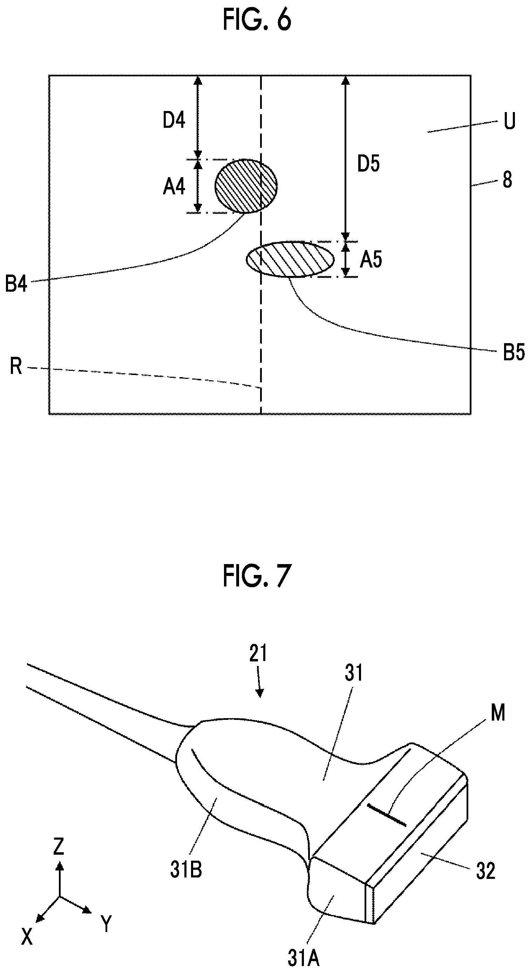

As illustrated in Fig. 6, for example, in a case where a plurality of blood vessel regions B4 and B5 each overlapping the highlight region R are extracted by the blood vessel extraction unit 9, the highlighting unit 12 can perform highlighting such that the plurality of blood vessel regions B4 and B5 extracted by the blood vessel extraction unit 9 have different colors or brightness from each other according to the depth or size of the blood vessel regions B4 and B5. In the example illustrated in Fig. 6, two blood vessel regions B4 and B5 overlap the linear highlight region R. A depth D4 of the blood vessel region B4 is shallower than a depth D5 of the blood vessel region B5, and a diameter A4 of the blood vessel region B4 is larger than a diameter A5 of the blood vessel region B5. In this case, the highlighting unit 12 can display the blood vessel region B4 and the blood vessel region B5 on the display device 8 such that the blood vessel region B4 and the blood vessel region B5 have colors defined in the order of depth or shallowness by comparing the depth D4 of the blood vessel region B4 and the depth D5 of the blood vessel region B5, for example.

Further, the highlighting unit 12 can display the blood vessel region B4 and the blood vessel region B5 on the display device 8 such that the blood vessel region B4 and the blood vessel region B5 have colors defined in a descending order or an ascending order of diameters by comparing the diameter A4 of the blood vessel region B4 and the diameter A5 of the blood vessel region B5, for example. Further, the highlighting unit 12 can display the blood vessel regions B4 and B5 on the display device 8 such that the brightness of the blood vessel region B4 having a relatively shallow depth is brighter than the brightness of the blood vessel region B5 having a relatively deep depth. Further, the highlighting unit 12 can display the blood vessel regions B4 and B5 on the display device 8 such that the brightness of the blood vessel region B4 having a relatively large diameter A4 is brighter than the brightness of the blood vessel region B5 having a relatively small diameter A5.

In this manner, the plurality of blood vessel regions B4 and B5 overlapping the highlight region R are highlighted to have different colors of brightness from each other according to the depth or size of the blood vessel regions B4 and B5 so that the plurality of blood vessel regions B4 and B5 overlapping the highlight region R can be highlighted to be distinguished from each other, and therefore the operator can intuitively and accurately grasp the blood vessel as the observation target by checking the ultrasound image U.

An example in which the highlight region setting unit 11 sets the highlight region R according to the information relating to the shape, placement position, number, and the like of the highlight region R, which is input by the operator through the input device 14 in advance, has been described, but the shape, placement position, and number of the highlight region R can be set according to the type of the ultrasound probe 21, for example.

For example, Fig. 7 illustrates an example of the ultrasound probe 21. In Fig. 7, the ultrasound probe 21 has a housing 31 that holds the transducer array 2 (not illustrated), and the housing 31 has an array housing portion 31A that houses the transducer array 2, and a grip portion 31B which is connected to the array housing portion 31A and is for the operator to grip the ultrasound probe 21. Here, for the description, a direction from the grip portion 31B toward the array housing portion 31A is referred to as a +Y direction, a width direction of the ultrasound probe 21 orthogonal to the Y direction is referred to as an X direction, and a thickness direction of the ultrasound probe 21 orthogonal to the X direction and the Y direction is referred to as a Z direction.

An acoustic lens 32 is disposed at an end portion of the array housing portion 31A in the +Y direction. Although not illustrated, a so-called acoustic matching layer is disposed on the transducer array 2, the acoustic lens 32 is disposed on the acoustic matching layer, and a plurality of transducers included in the transducer array 2 are arranged along the X direction. A linear guide marker M extending along the Y direction is added to the outer peripheral portion of the array housing portion 31A. The guide marker M can be used as a guide in a case where the operator brings the ultrasound probe 21 into contact with the subject.

In this manner, in a case where the guide marker M is added to the housing 31 of the ultrasound probe 21, for example, the placement position of the highlight region R in the ultrasound image U can be decided in association with the placement position of the guide marker M in an arrangement direction of the plurality of transducers in the transducer array 2. In this manner, the operator can observe the blood vessel in the subject while associating the guide marker M added to the housing 31 of the ultrasound probe 21 with the position of the highlight region R in the ultrasound image U, and therefore it is possible to more accurately grasp the blood vessel as the observation target by observing the ultrasound image U.

In the example illustrated in Fig. 7, one guide marker M is added to the housing 31 of the ultrasound probe 21, but in a case where two or more guide markers M are added, it is desirable to set the highlight regions R of which the number corresponds to the number of guide markers M.

For example, the highlight region setting unit 11 can automatically decide the shape, placement position, number of the highlight region R according to the type of the ultrasound probe 21, for example, and can set the highlight region R according to the decided shape, placement position, number of the highlight region R. For example, although not illustrated, the ultrasound diagnostic apparatus 1 can comprise a probe type discrimination unit that automatically reads the type of the ultrasound probe 21 in a case where the ultrasound probe 21 is connected to the ultrasound diagnostic apparatus 1, and the highlight region setting unit 11 can automatically decide the shape, placement position, number of the highlight region R according to the type of the ultrasound probe 21 read by the probe type discrimination unit. In this manner, since the highlight region R corresponding to the guide marker M added to the housing 31 of the ultrasound probe 21 can be automatically set regardless of the type of the ultrasound probe 21, the operator can more accurately grasp the blood vessel as the observation target while reducing the effort to decide the shape, placement position, and number of the highlight region R.

The highlight region setting unit 11 can prevent the set highlight region R from being displayed on the display device 8, and can also display the set highlight region R on the display device 8 such that the set highlight region R is superimposed on the ultrasound image U. In a case where the highlight region R is displayed on the display device 8, since the operator can move the ultrasound probe 21 on the body surface of the subject by using the highlight region R displayed by being superimposed on the ultrasound image U as a guide, it is possible to more accurately grasp the blood vessel as the observation target while easily adjusting the position, inclination, and the like of the ultrasound probe 21. Here, moving the ultrasound probe 21 includes moving the ultrasound probe 21 in parallel and tilting the ultrasound probe 21.

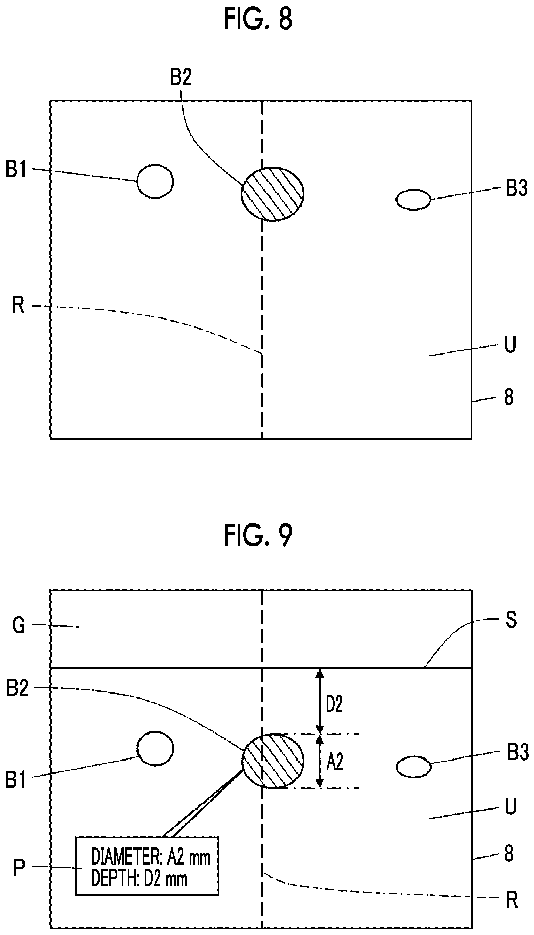

As illustrated in Fig. 8, for example, in a case where the highlight region R is displayed on the display device 8 and the blood vessel region B2 extracted by the blood vessel extraction unit 9 overlaps the highlight region R, the highlight region setting unit 11 can display only a part of the highlight region R, which does not overlap the blood vessel region B2, on the display device 8. In this manner, since the highlight region R is prevented from hiding the blood vessel region B2, the operator can more accurately grasp the blood vessel as the observation target.

In general, a procedure is known in which a gel layer for so-called ultrasonography is applied to the body surface of the subject and the inside of the subject is observed in a state where the ultrasound probe is in contact with the applied gel layer. Here, the gel layer is composed of a highly viscous liquid such as a so-called polymer-absorbing gel. In general, the reflection and attenuation of the ultrasonic waves are likely to occur in a case where an air layer is present between the ultrasound probe and the body surface of the subject, but the gel layer can sufficiently transmit the ultrasonic waves and fill the gap between the body surface of the subject and the ultrasound probe, and therefore it is possible to reduce the reflection and attenuation of the ultrasonic waves between the ultrasound probe and the body surface of the subject.

In this manner, in a case where the ultrasound image U is generated in a state where the ultrasound probe 21 is in contact with the gel layer applied to the body surface of the subject, for example, as illustrated in Fig. 9, a gel layer G and a body surface S of the subject are included in the ultrasound image U. In this case, by analyzing the ultrasound image U, the blood vessel information acquisition unit 10 can recognize the body surface S of the subject, and measure the shortest distance from the recognized body surface S of the subject to the blood vessel region B2 in the depth direction as the depth D2 of the blood vessel region B2. In this case, the blood vessel information acquisition unit 10 can recognize the body surface S of the subject by using, for example, a known algorithm such as template matching, a machine learning method, a general image recognition method using deep learning or the like.

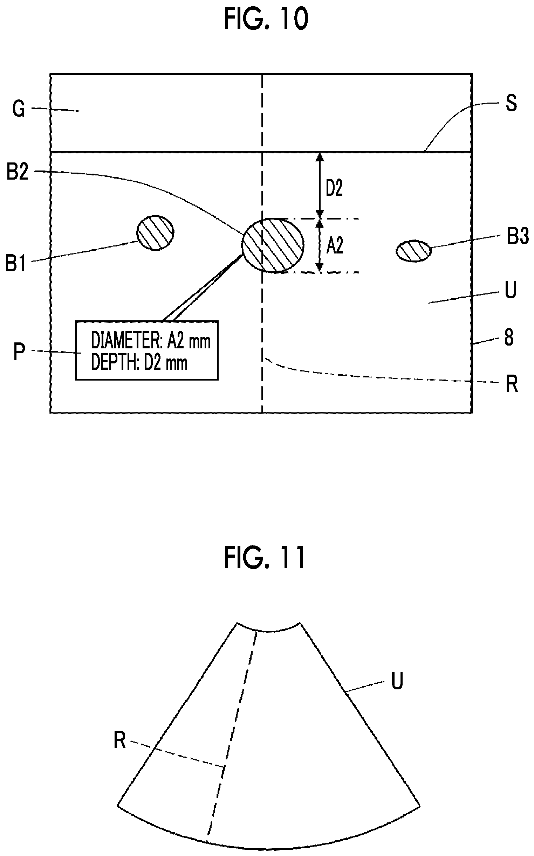

Further, as illustrated in Fig. 10, the highlighting unit 12 can display the blood vessel information panel P representing the blood vessel information of only the blood vessel region B2 overlapping the highlight region R while highlighting all the blood vessel regions B1, B2, and B3 extracted by the blood vessel extraction unit 9 such that the blood vessel regions B1, B2, and B3 have the same color and brightness as each other. In this manner, by searching for the presence of the blood vessel region in a wide range and displaying the blood vessel information of only the blood vessel region of interest, it is possible to achieve both prevention of overlooking of the blood vessel region and efficiency of grasping the blood vessel information.

In a case where the blood vessel region B2 extracted by the blood vessel extraction unit 9 overlaps the highlight region R, the device control unit 13 can set an imaging condition so that the blood vessel region B2 is clearly depicted in the ultrasound image U, and can control the transmission and reception circuit 5 and the image generation unit 6 on the basis of the set imaging condition. The imaging condition includes, for example, a condition for the transmission and reception circuit 5, and a condition for the image generation unit 6. Examples of the condition for the transmission and reception circuit 5 include the transmission frequency of ultrasonic waves, the focal position, and the display depth, and examples of the condition for the image generation unit 6 include a sound speed, a detection condition, gain, dynamic range, a gradation curve, speckle suppression strength, and an edge enhancement degree.

In this manner, the imaging condition is set according to the blood vessel region B2 overlapping the highlight region R so that the blood vessel region B2 is more clearly depicted, and therefore, the operator can more intuitively and accurately grasp the blood vessel as the observation target.

In a case where the ultrasound probe 21 connected to the ultrasound diagnostic apparatus 1 is a so-called convex type or sector type ultrasound probe in which a plurality of transducers constituting the transducer array 2 are curvedly arranged, as illustrated in Fig. 11, the highlight region R can be set to have a shape extending along a depth direction by using a direction along a scan line as the depth direction. In this manner, even in a case where the shape of the highlight region R is set to a shape corresponding to the arrangement direction of the plurality of transducers of the transducer array 2, the operator can more intuitively and accurately grasp the blood vessel as the observation target by checking the generated ultrasound image U.

### Second Embodiment

In general, a procedure of inserting an insertion object such as a so-called puncture needle and a catheter into the blood vessel of the subject while observing the inside of the subject using the ultrasound diagnostic apparatus is known, but the highlighting unit 12 can highlight the blood vessel region B2 overlapping the highlight region R on the display device 8 by further adding the path of the insertion object inserted into the subject.

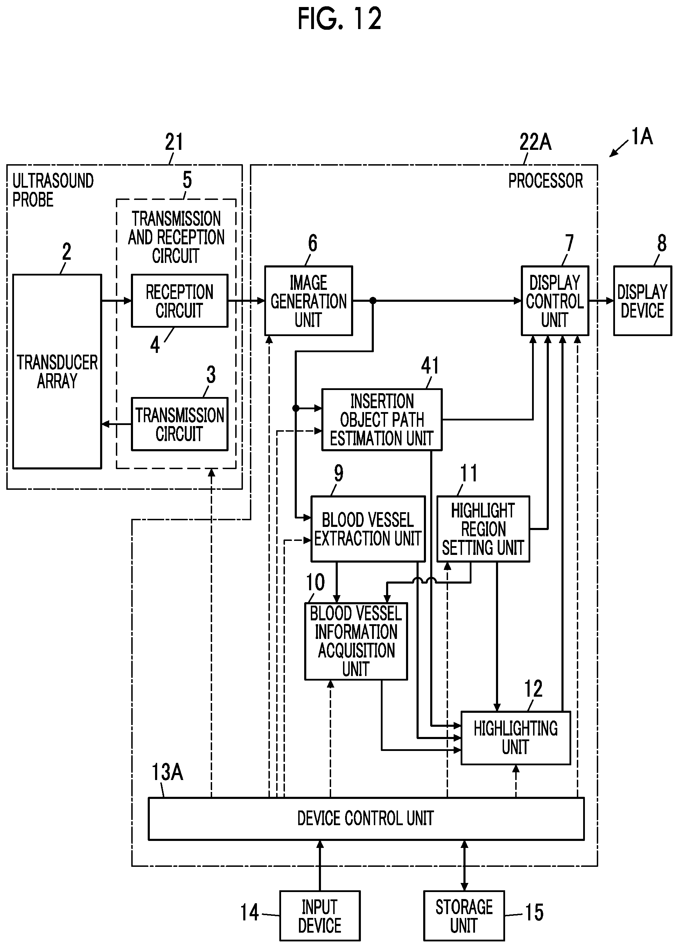

Fig. 12 illustrates a configuration of an ultrasound diagnostic apparatus 1A according to a second embodiment of the present invention. The ultrasound diagnostic apparatus 1A is obtained by adding an insertion object path estimation unit 41 to the ultrasound diagnostic apparatus 1 of the first embodiment illustrated in Fig. 1, comprising a device control unit 13A instead of the device control unit 13, and comprising a processor 22A instead of the processor 22. In the ultrasound diagnostic apparatus 1A, the insertion object path estimation unit 41 is connected to the image generation unit 6, and the display control unit 7 and the highlighting unit 12 are connected to the insertion object path estimation unit 41.

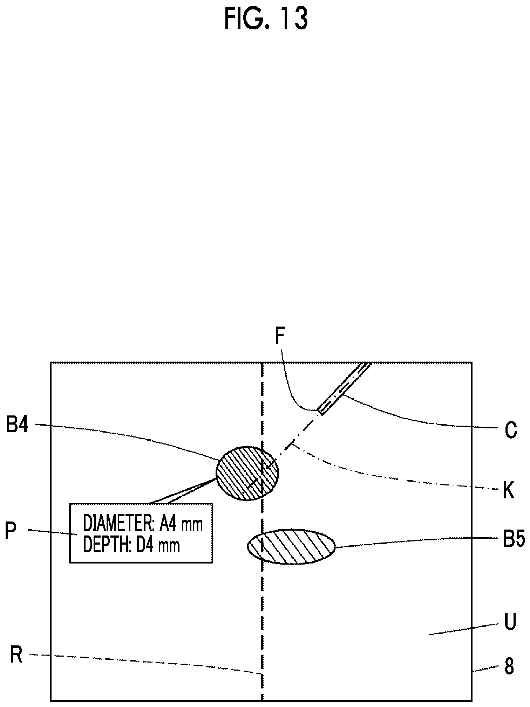

As illustrated in Fig. 13, the insertion object path estimation unit 41 detects an insertion object C by analyzing the ultrasound image U generated by the image generation unit 6, and estimates a path K of the detected insertion object C. The insertion object path estimation unit 41 can detect the insertion object C by using, for example, a known algorithm such as template matching, a machine learning method, a general image recognition method using deep learning or the like. For example, in a case where the insertion object C depicted in the ultrasound image U has a rod shape extending in one direction, the insertion object path estimation unit 41 can estimate the linear path K extending from a distal end F of the insertion object C along a direction in which the insertion object C extends. For example, the insertion object path estimation unit 41 can detect a trajectory of the distal end F of the insertion object C by analyzing the ultrasound images U of a plurality of frames consecutively generated by the image generation unit 6, and estimate the path K of the insertion object C on the basis of the detected trajectory.

In a case where there are the plurality of blood vessel regions B4 and B5 overlapping the highlight region R, the highlighting unit 12 highlights, among the plurality of blood vessel regions B4 and B5, the blood vessel region B4, which overlaps both the highlight region R and the path K of the insertion object C estimated by the insertion object path estimation unit 41, on the display device 8 such that the blood vessel region B4 is distinguished from the blood vessel region B5 overlapping only the highlight region R. For example, the highlighting unit 12 can perform highlighting such that the blood vessel region B4 and the blood vessel region B5 have different colors or brightness from each other. Further, the highlighting unit 12 can display only the blood vessel information relating to the blood vessel region B4 overlapping both the highlight region R and the estimated path K of the insertion object C, on the display device 8, among the blood vessel regions B4 and B5 extracted by the blood vessel extraction unit 9, for example. In the example illustrated in Fig. 13, only the blood vessel information panel P including the diameter A4 and the depth D4 as the blood vessel information of the blood vessel region B4 among the blood vessel regions B4 and B5 is superimposed on the ultrasound image U to be displayed on the display device 8.

From the above, with the ultrasound diagnostic apparatus 1A according to the second embodiment of the present invention, the path K of the insertion object C inserted into the subject is estimated, and the blood vessel region B4 overlapping both the highlight region R and the estimated path K of the insertion object C is highlighted on the display device 8 so as to be distinguished from the blood vessel region B5 overlapping only the highlight region R. Therefore, the operator can accurately grasp the blood vessel into which the insertion object C is to be inserted by checking the ultrasound image U, and it is possible to improve the accuracy of inserting the insertion object C into the blood vessel.

The insertion object path estimation unit 41 can prevent the estimated path K of the insertion object C from being displayed on the display device 8, and can also display the estimated path K of the insertion object C on the display device 8 such that the estimated path K is superimposed on the ultrasound image U. In a case where the path K of the insertion object C is displayed on the display device 8, since the operator can insert the insertion object C into the subject while checking the displayed path K of the insertion object C, the operator can more accurately grasp the blood vessel into which the insertion object C is to be inserted, and it is possible to further improve the accuracy of inserting the insertion object C into the blood vessel.

### Third Embodiment

In the first embodiment, the highlighting unit 12 displays the blood vessel information of the blood vessel region B2 overlapping the highlight region R, on the display device 8, the blood vessel information being acquired by the blood vessel information acquisition unit 10, but the highlighting unit 12 can display the blood vessel information relating to the blood vessel region B2 on the display device 8 only in a case where the ultrasound probe 21 is stationary, for example.

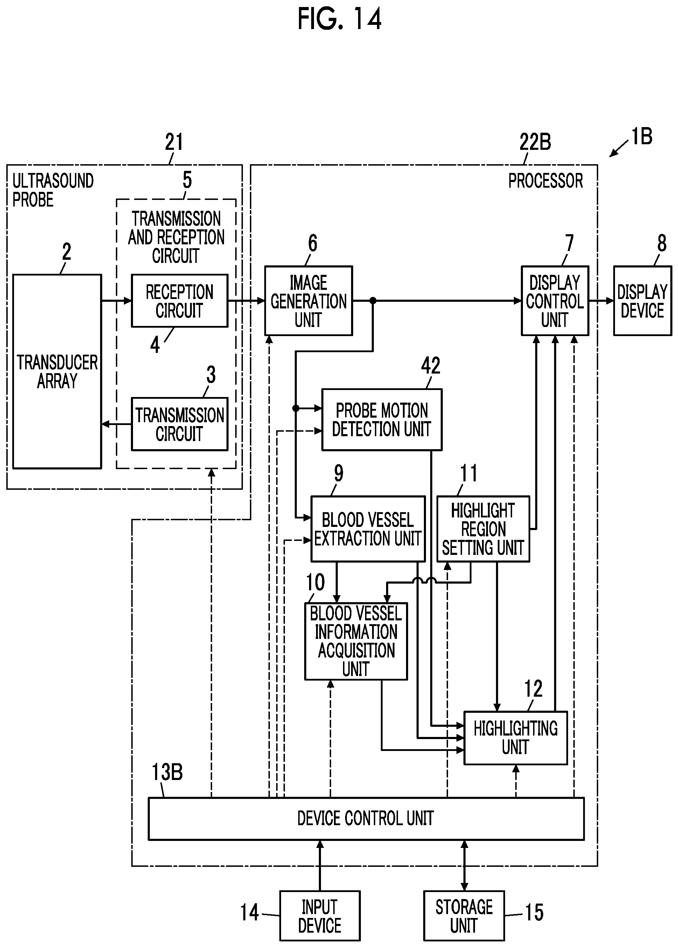

Fig. 14 illustrates a configuration of an ultrasound diagnostic apparatus 1B according to a third embodiment of the present invention. The ultrasound diagnostic apparatus 1B according to the third embodiment is obtained by adding a probe motion detection unit 42 to the ultrasound diagnostic apparatus 1 of the first embodiment illustrated in Fig. 1, comprising a device control unit 13B instead of the device control unit 13, and comprising a processor 22B instead of the processor 22. In the ultrasound diagnostic apparatus 1B, the probe motion detection unit 42 is connected to the image generation unit 6, and the highlighting unit 12 is connected to the probe motion detection unit 42.

The probe motion detection unit 42 detects the motion of the ultrasound probe 21, and determines whether the ultrasound probe 21 is stationary on the basis of the detected motion of the ultrasound probe 21. In this case, the probe motion detection unit 42 detects the amount of change of the image between the consecutive frames as the motion of the ultrasound probe 21 by analyzing the ultrasound images U of the plurality of frames consecutively generated by the image generation unit 6, determines that the ultrasound probe 21 is stationary in a case where the detected amount of change of the image is equal to or less than a predetermined threshold value, and determines that the ultrasound probe 21 is moving in a case where the detected amount of change of the image is greater than the predetermined threshold value. Here, as the amount of change of the image, for example, a distance that the image has moved and an angle that the image has rotated between the ultrasound images U of two consecutive frames can be used.

Only in a case where the probe motion detection unit 42 determines that the ultrasound probe 21 is stationary, the highlighting unit 12 displays the blood vessel information relating to the blood vessel region B2 overlapping the highlight region R on the display device 8.

From the above, with the ultrasound diagnostic apparatus 1B according to the third embodiment of the present invention, the motion of the ultrasound probe 21 is detected, and the blood vessel information relating to the blood vessel region B2 overlapping the highlight region R is displayed on the display device 8 only in a case where it is determined that the ultrasound probe 21 is stationary on the basis of the detected motion of the ultrasound probe 21. Therefore, the ultrasound probe 21 can be moved while checking the entire ultrasound image U even in a case where the blood vessel region B2 overlaps the highlight region R, and it is possible to easily check the blood vessel information of the blood vessel as the observation target by making the ultrasound probe 21 stationary.

It is described that the probe motion detection unit 42 detects the motion of the ultrasound probe 21 by analyzing the ultrasound image U generated by the image generation unit 6, but the method of detecting the motion of the ultrasound probe 21 is not limited thereto. For example, although not illustrated, the ultrasound diagnostic apparatus 1B can comprise a probe motion sensor including a sensor such as an acceleration sensor, a gyro sensor, a magnetic sensor, and a global positioning system (GPS). In this case, the probe motion detection unit 42 can detect the motion of the ultrasound probe 21 on the basis of the signal detected by the probe motion sensor.

Further, it is described that the form of the third embodiment is applied to the ultrasound diagnostic apparatus 1 of the first embodiment, but the form of the third embodiment can be similarly applied to the ultrasound diagnostic apparatus 1A of the second embodiment.

### Fourth Embodiment

The ultrasound diagnostic apparatus 1 of the first embodiment has the configuration in which the display device 8, the input device 14, and the ultrasound probe 21 are directly connected to the processor 22, but, for example, the display device 8, the input device 14, the ultrasound probe 21, and the processor 22 can be indirectly connected to each other via the network.

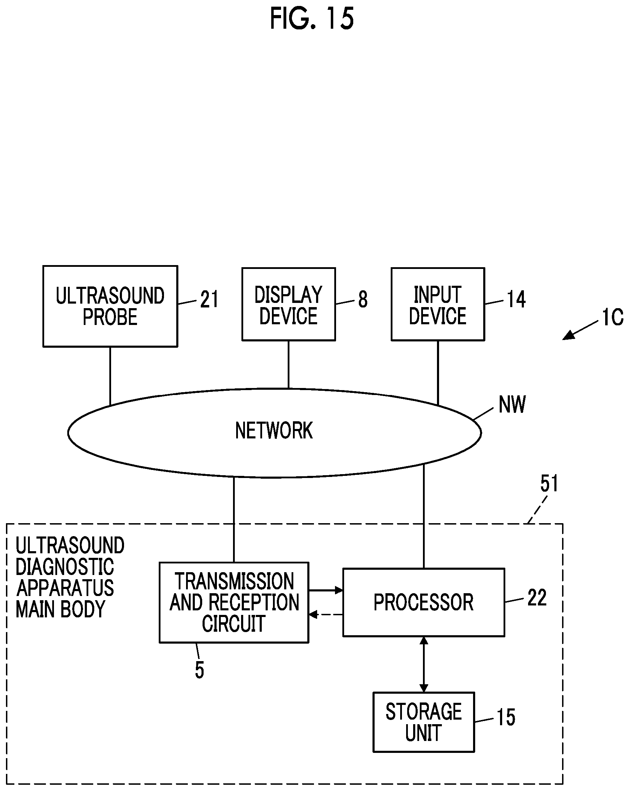

As illustrated in Fig. 15, in an ultrasound diagnostic apparatus 1C according to a fourth embodiment, the display device 8, the input device 14, and the ultrasound probe 21 are connected to an ultrasound diagnostic apparatus main body 51 via a network NW. The ultrasound diagnostic apparatus main body 51 is obtained by excluding the display device 8, the input device 14, and the ultrasound probe 21 in the ultrasound diagnostic apparatus 1 of the first embodiment illustrated in Fig. 1, and is constituted by the transmission and reception circuit 5, the storage unit 15, and the processor 22.

Even in a case where the ultrasound diagnostic apparatus 1B has such a configuration, similarly to the ultrasound diagnostic apparatus 1 of the first embodiment, since the highlight region R is set on the generated ultrasound image U and the blood vessel region B2 overlapping the highlight region R is highlighted on the display device 8, the operator can intuitively and accurately grasp the blood vessel region B2 as the observation target by checking the ultrasound image U.

Further, since the display device 8, the input device 14, and the ultrasound probe 21 are connected to the ultrasound diagnostic apparatus main body 51 via the network NW, the ultrasound diagnostic apparatus main body 51 can be used as a so-called remote server. Thereby, for example, since the operator can perform a diagnosis of the subject by preparing the display device 8, the input device 14, and the ultrasound probe 21 at the operator's hand, it is possible to improve the convenience in a case of the ultrasound diagnosis.

Further, in a case where a portable thin computer, for example, a so-called tablet, is used as the display device 8 and the input device 14, it is possible for the operator to more easily perform the ultrasound diagnosis of the subject, and it is possible to further improve the convenience in a case of the ultrasound diagnosis.

The display device 8, the input device 14, and the ultrasound probe 21 are connected to the ultrasound diagnostic apparatus main body 51 via the network NW, but in this case, the display device 8, the input device 14, and the ultrasound probe 21 may be connected to the network NW in a wired manner or in a wireless manner.

Further, it is described that the form of the fourth embodiment is applied to the ultrasound diagnostic apparatus 1 of the first embodiment, but the form of the fourth embodiment can be similarly applied to the ultrasound diagnostic apparatus 1A of the second embodiment and the ultrasound diagnostic apparatus 1B of the third embodiment.

### Explanation of References

1, 1A, 1B, 1C: ultrasound diagnostic apparatus
2: transducer array
3: transmission circuit
4: reception circuit
5: transmission and reception circuit
6: image generation unit
7: display control unit
8: display device
9: blood vessel extraction unit
10: blood vessel information acquisition unit
11: highlight region setting unit
12: highlighting unit
13, 13A, 13B: device control unit
14: input device
15: storage unit
21: ultrasound probe
22, 22A, 22B: processor
23: amplification unit
24: AD conversion unit
25: beam former
26: signal processing unit
27: DSC
28: image processing unit
31: housing
31A: array housing portion
31B: grip portion
32: acoustic lens
41: insertion object path estimation unit
42: probe motion detection unit
51: ultrasound diagnostic apparatus main body
A2, A4, A5: diameter
B1, B2, B3, B4, B5: blood vessel region
C: insertion object
D2, D4, D5: depth
F: distal end
G: gel layer
K: path
M: guide marker
NW: network
P: blood vessel information panel
R: highlight region
S: body surface
U: ultrasound image

## Claims

1. An ultrasound diagnostic apparatus that displays a blood vessel of a subject on an ultrasound image, the ultrasound diagnostic apparatus comprising:
a transducer array (2);
a transmission and reception circuit configured to cause the transducer array to transmit an ultrasound beam toward the subject, and to process a reception signal output from the transducer array that has received an ultrasound echo from the subject to generate a sound ray signal;
an image generation unit (6) configured to generate the ultrasound image on the basis of the sound ray signal generated by the transmission and reception circuit;
a display device (8) arranged to display the ultrasound image generated by the image generation unit;
a blood vessel extraction unit (9) configured to extract blood vessel regions by analyzing the ultrasound image generated by the image generation unit;
a highlight region setting unit (11) configured to set at least one highlight region extending in a depth direction of the ultrasound image;
a highlighting unit (12) configured to highlight the blood vessel region which is extracted by the blood vessel extraction unit and overlaps the at least one highlight region set by the highlight region setting unit, on the display device; and
an insertion object path estimation unit (41) configured to detect an insertion object and to estimate a path of the detected insertion object by analyzing the ultrasound image generated by the image generation unit in a case where the insertion object is inserted into the blood vessel of the subject,
wherein in a case where a plurality of the blood vessel regions each overlapping the highlight region are detected by the blood vessel extraction unit and the insertion object is detected by the insertion object path estimation unit, the highlighting unit performs highlighting such that the blood vessel region overlapping both the highlight region and the path of the insertion object estimated by the insertion object path estimation unit, and the blood vessel region overlapping only the highlight region have different colors or brightness from each other.

2. The ultrasound diagnostic apparatus according to claim 1, further comprising:
a blood vessel information acquisition unit (10) configured to acquire blood vessel information including at least one of a diameter or a depth of the blood vessel region overlapping the highlight region by analyzing the ultrasound image generated by the image generation unit,
wherein the highlighting unit displays the blood vessel information acquired by the blood vessel information acquisition unit on the display device in association with the blood vessel region overlapping the highlight region.

3. The ultrasound diagnostic apparatus according to claim 2,
wherein the blood vessel information acquisition unit (10) configured to further acquire information representing whether the blood vessel region belongs to an artery or a vein, as the blood vessel information.

4. The ultrasound diagnostic apparatus according to claim 2 or 3,
wherein in a case where a plurality of the blood vessel regions are extracted by the blood vessel extraction unit, the highlighting unit (12) highlights the plurality of blood vessel regions extracted by the blood vessel extraction unit on the display device (8) such that the blood vessel regions have the same color and brightness as each other, and displays the blood vessel information acquired by the blood vessel information acquisition unit on the display device in association with the blood vessel region overlapping the highlight region among the plurality of blood vessel regions.

5. The ultrasound diagnostic apparatus according to any one of claims 1 to 3,
wherein in a case where a plurality of the blood vessel regions are extracted by the blood vessel extraction unit, the highlighting unit (12) highlights each of the plurality of blood vessel regions extracted by the blood vessel extraction unit on the display device (8), and highlights, among the plurality of blood vessel regions, the blood vessel region overlapping the highlight region and the blood vessel region not overlapping the highlight region in different formats.

6. The ultrasound diagnostic apparatus according to claim 5,
wherein the highlighting unit (12) is configured to perform highlighting such that, among the plurality of blood vessel regions extracted by the blood vessel extraction unit, the blood vessel region overlapping the highlight region and the blood vessel region not overlapping the highlight region have different colors or brightness from each other.

7. The ultrasound diagnostic apparatus according to any one of claims 1 to 3,
wherein in a case where a plurality of the blood vessel regions each overlapping the highlight region are extracted by the blood vessel extraction unit, the highlighting unit (12) performs highlighting such that the plurality of blood vessel regions extracted by the blood vessel extraction unit have different colors or brightness from each other according to a depth or size of the blood vessel regions.

8. The ultrasound diagnostic apparatus according to claim 2 or 3, further comprising:
an ultrasound probe (21) including the transducer array; and
a probe motion detection unit (42) configured to detect a motion of the ultrasound probe, and determines whether the ultrasound probe is stationary on the basis of the detected motion of the ultrasound probe,
wherein the highlighting unit displays the blood vessel information acquired by the blood vessel information acquisition unit on the display device only in a case where the probe motion detection unit determines that the ultrasound probe is stationary.

9. The ultrasound diagnostic apparatus according to any one of claims 1 to 8, further comprising:
a device control unit (13) configured to set an imaging condition such that the blood vessel region is clearly depicted in the ultrasound image, and to control the transmission and reception circuit and the image generation unit on the basis of the set imaging condition, in a case where the blood vessel region extracted by the blood vessel extraction unit overlaps the highlight region.

10. The ultrasound diagnostic apparatus according to any one of claims 1 to 9,
wherein the highlight region setting unit displays the set highlight region on the display device.

11. The ultrasound diagnostic apparatus according to claim 10,
wherein the highlight region setting unit displays only a part of the highlight region, which does not overlap the blood vessel region, on the display device in a case where the blood vessel region extracted by the blood vessel extraction unit overlaps the highlight region.

12. A control method of an ultrasound diagnostic apparatus that displays a blood vessel of a subject on an ultrasound image, the control method comprising:
causing a transducer array (2) to transmit an ultrasound beam toward the subject, and processing a reception signal output from the transducer array that has received an ultrasound echo from the subject to generate a sound ray signal;
generating the ultrasound image on the basis of the generated sound ray signal;
displaying the generated ultrasound image on a display device (8);
extracting blood vessel regions by analyzing the generated ultrasound image;
setting at least one highlight region extending in a depth direction of the ultrasound image;
detecting an insertion object and estimating a path of the detected insertion object by analyzing the ultrasound image in a case where the insertion object is inserted into the blood vessel of the subject; and
in a case where a plurality of the blood vessel regions each overlapping the highlight region are detected and the insertion object is detected, performing highlighting on the display device such that the blood vessel region overlapping both the highlight region and the path of the estimated insertion object, and the blood vessel region overlapping only the highlight region have different colors or brightness from each other.
